# EUROPÄISCHE PATENTSCHRIFT

(11) **EP 0 796 634 B1**
(45) Veröffentlichungstag und Bekanntmachung des Hinweises auf die Patenterteilung: **16.11.2005**
(21) Anmeldenummer: 97250090.4
(22) Anmeldetag: 20.03.1997
(51) Int. Cl.: A61N 1/05

(54) **Implantierbare Stimulationselektrode**
Implantable stimulation electrode
Electrode de stimulation implantable

(30) Priorität: 21.03.1996 DE 19613002; 19.07.1996 DE 19630563
(43) Veröffentlichungstag der Anmeldung: 24.09.1997
(73) Patentinhaber: BIOTRONIK Mess- und Therapiegeräte GmbH & Co Ingenieurbüro Berlin, 12359 Berlin (DE); Max-Planck-Gesellschaft zur Förderung der Wissenschaften e.V. Berlin, 80539 München (DE)
(72) Erfinder: Bolz, Armin, Dr. habil., 91058 Erlangen (DE); Fröhlich, Ronald, Dr., 91056 Erlangen (DE); Stelzle, Martin, Dr., 72762 Reutlingen (DE); Schmitt, Johannes, 65520 Bad Camberg (DE); Diederich, Anke, 55122 Mainz (DE); Cassier, Thorsten, 65510 Idstein (DE); Wagner, Roland, Dr., 12437 Berlin (DE)
(74) Vertreter: Eisenführ, Speiser & Partner

(56) Entgegenhaltungen:
- EP-A- 0 633 031
- US-A- 4 336 811
- US-A- 4 542 752
- US-A- 4 784 160
- US-A- 4 921 723
- US-A- 5 103 837

## Beschreibung

Die Erfindung betrifft eine implantierbare Stimulationselektrode der im Oberbegriff des Anspruchs 1 angegebenen Art.

Elektroden zur Stimulation von Körpergewebe mittels elektrischer Reize sind in großer Vielgestalt bekannt. Obgleich zur Übertragung elektrischer Ströme an lebendes Gewebe - ebenso wie an andere Objekte - aufgrund der erforderlichen guten Leitfähigkeit metallische Werkstoffe bei weitem am häufigsten eingesetzt werden, sind auch Elektroden bekannt, bei denen Elemente aus leitfähigen Polymeren vorgesehen sind - vgl. etwa US 5 080 099, worin Hautelektroden mit Hydrogel-Kontaktelementen als Stimulationselektroden für einen externen Defibrillator und/oder Herzschrittmacher beschrieben werden.

Im Zuge der schnellen Entwicklung bei der Technik implantierbarer Herzschrittmacher und Defibrillatoren ist auch der Verfeinerung des Aufbaus und der optimalen Wahl des Materials für die zugehörigen implantierbaren Elektroden großes Augenmerk geschenkt worden. Eine Zusammenfassung der hierbei zu beachtenden physikochemischen Gesetzmäßigkeiten sowie Hinweise auf wesentliche Entwicklungen sowohl hinsichtlich Schrittmacherelektroden als auch hinsichtlich biokompatibler Werkstoffe aus dem Blickwinkel der Schrittmachertechnik bis Anfang der neunziger Jahre (einschließlich umfangreicher Quellenangaben) finden sich in M. Schaldach: Electrotherapy of the Heart - Technical Aspects in Cardiac Pacing, Springer 1992, S. 145-190.

Für den Gebrauchswert einer implantierbaren Stimulationselektrode - insbesondere einer solchen, die für den Langzeiteinsatz an einem Gewebestimulator mit einer erschöpfbaren Energiequelle gedacht ist und daher zu minimalem Energieverbrauch beitragen muß - sind eine hohe Elektrodenkapazität und damit niedrige Elektrodenimpedanz und ein möglichst hoher Grad an Biokompatibilität von herausragender Bedeutung.

In diesem Sinne hochentwickelte implantierbare Stimulationselektroden sind etwa in EP 0 453 117 A1 oder in WO 93/02739 beschrieben. Die erstgenannte Druckschrift beschreibt einen mehrschichtigen Elektrodenaufbau mit einem aus Faser- bzw. Drahtmaterial gepreßten Platin-Grundkörper, einer Haftschicht, einer Pt-, C- oder Al-Texturierungsschicht mit rauher Oberfläche und einer katalytisch wirkenden Pt- oder Pt/C-Deckschicht. In der letztgenannten Druckschrift ist eine wesentlich einfacher aufgebaute Stimulationselektrode beschrieben, die infolge einer inerten Beschichtung mit fraktaler Oberflächenstruktur eine sehr große aktive Oberfläche aufweist. Diese Elektrode besteht in einer vorteilhaften Ausführung aus einem Titan-Grundkörper mit einer Iridium-, Iridiumnitrid- oder Iridiumoxidbeschichtung.

Auch bei weitgehend optimierten Elektroden der letztgenannten Art tritt nach der Implantation eine zeitweilige Erhöhung der Reizschwelle aufgrund von Gewebsirritationen auf, die in dieser Phase zu einem erhöhten Energieverbrauch des Stimulators führt und u.U. - je nach Gerätetyp - aufwendige Einstellungskorrekturen erfordert.

Aus US 4 552 635 ist eine in eine offenzellige, mit einer speziellen Flüssigkeit gefüllte Polymermatrix eingekapselte Referenzelektrode bekannt, die für Meßanordnungen zur invivo-Bestimmung des pH-Wertes gedacht ist. Diese Elektrode ist keine Stimulationelektrode und als solche auch nicht einsetzbar.

EP 0 633 031 A1 beschreibt eine anti-fouling-Beschichtung für medizinische Geräte, unter anderen auch als Beschichtung für eine Elektrodenoberfläche eines Schrittmachers. Durch die Beschichtung soll unkontrollierten Gewebswucherungen vorgebeugt werden und eine dünne Endothelbenetzung initiiert werden. Die Beschichtung wird durch Eintauchen der Elektrode in eine Polyethylenglycol-Lösung erzeugt.

Der Erfindung liegt die Aufgabe zugrunde, eine implantierbare Stimulationselektrode der eingangs genannten Gattung anzugeben, mit der Gewebsirritationen nach der Implantation und speziell ein damit einhergehender Reizschwellenanstieg vermieden werden können.

Diese Aufgabe wird durch eine Stimulationselektrode mit den Merkmalen des Anspruchs 1 gelöst.

Die Erfindung schließt den Gedanken ein, auf der Oberfläche einer implantierbaren Stimulationselektrode eine organische Schicht vorzusehen, die die unspezifische Adsorption biologischer Makromoleküle verhindert oder zumindest entscheidend reduziert und wahlweise spezifisch funktionalisiert oder funktionalisierbar ist. Unter einer "organischen Schicht" wird im weiteren auch ein solche mit Siliziumatomen subsumiert, wie sie etwa durch Reaktion mit Silanen gebildet werden kann. Die Erfindung geht aus von einer Stimulationselektrode, bei der eine im wesentlichen die gesamte äußere Oberfläche der Stimulationselektrode bildende, dünne, spezifisch funktionalisierte organische Beschichtung vorgesehen ist, die aufgrund irreversibler Physisorption oder kovalenter chemischer Bindung an der darunterliegenden Oberfläche fest haftet. Erfindungsgemäß ist vorgesehen, dass die organische Beschichtung einen durch sukzessive Adsorption eines anionischen und eines kationischen Polyelektrolyten gebildeten mehrschichtigen Aufbau aufweist.

Eine praktisch potentiell bedeutsame zusätzliche Funktionalisierung besteht darin, daß die organische Schicht Sondenmoleküle (z.B. Enzyme) aufweist derart, daß die Stimulationselektrode als Biosensorelektrode wirken kann.

Eine weitere wichtige Funktionalisierung besteht darin, daß die organische Schicht einen medizinischen Wirkstoff, insbesondere eine entzündungshemmendes Medikament, aufweist, der aus der organischen Schicht diffusions- oder lösungsgesteuert austragbar ist. Insbesondere kann der medizinische Wirkstoff im wesentlichen zwischen Teilschichten des mehrschichtigen Aufbaus eingebettet sein.

Die organische Schicht ist ultradünn, d.h. ihre Schichtdicke liegt im Bereich zwischen 1 und 100 nm, in bestimmten Ausführungen (etwa als Polyelektrolyt-Multischicht) bevorzugt im Bereich zwischen 3 und 50 nm.

Zur Gewährleistung vorteilhafter elektrischer Eigenschaften. speziell einer geringen Beeinflussung der hohen Phasengrenzkapazität hochentwickelter Stimulationselektroden auch bei größeren Schichtdicken im o.g. Bereich wird die organische Schicht in einer vorteilhaften Ausführung so ausgebildet, daß sie eine relative Dielektrizitätskonstante von größer als 100, insbesondere von größer als 300, aufweist. Bei sehr geringen Schichtdicken können auch Schichten mit relativ niedriger Dielektrizitätskonstante eingesetzt werden.

Die organische Beschichtung hat einen durch sukzessive Adsorption eines anionischen und eines kationischen Polyelektrolyten gebildeten mehrschichtigen Aufbau, wobei sie insbesondere zwischen 10 und 50 Paare (Aufbau A-B-A-B-...) eines anionischen und eines kationischen Schichtbildners aufweist.

In von den Erfindern genauer untersuchten, in zweckmäßiger Weise unter Nutzung kommerziell leicht erhältlicher Verbindungen gebildeten Schichtaufbauten werden als Schichtbildner Polystyrensulfonat (Kurzbezeichnung PSS) bzw. Polystyrensulfonat-Natriumsalz (Na-PSS) oder Polyvinylsulfonat oder ein Polyvinylsulfonat-Kaliumsalz und als anionischen Polyelektrolyt (Verbindung A) und Poly-4-vinylbenzyl(N,N-diethyl-N-methyl)ammoniumiodid oder ein Polyallylamin bzw. Polyallylamin-Hydrochlorid (PAH) als kationischen Polyelektrolyt (Verbindung B) eingesetzt. In einer vorteilhaften Tripel-Schicht kann als Verbindung C

Poly-(1-(4-(3-carboxy-4-hydroxyphenylazo)benzensulfonamid)-1,2-ethandiyl)-Natriumsalz (PAZO) eingesetzt werden.

Die äußerste Schicht kann insbesondere ein ungeladenes Polymeres, beispielsweise Polyethylenoxid oder Bisamino-Polyethylenglykol, aufweisen.

Gute Haftung der organischen Schicht und gleichzeitig eine hohe Phasengrenzkapazität sind in vorteilhafter Weise mit einer Ausführung der Stimulationselektrode erreichbar, bei der die unter der organischen Schicht liegende Oberfläche eine ausgeprägte Oberflächenrauhigkeit bzw. -porosität aufweist, so daß die effektive Physisorptions-Oberfläche um mindestens eine Größenordnung größer als die sich aus der geometrischen Grundform der Stimulationselektrode ergebende Oberfläche ist. Besonders bevorzugt ist dabei eine fraktale Oberflächengeometrie, mit der die effektive Physisorptions-Oberfläche um zwei bis drei Größenordnungen gegenüber der sich aus der geometrischen Grundform der Stimulationselektrode ergebenden Oberfläche vergrößert werden kann.

Ein Elektrodenaufbau mit vorteilhaften Eigenschaften ergibt sich nach den Untersuchungen der Erfinder zudem, wenn zwischen dem metallischen Trägerkörper und der organischen Schicht eine nichtmetallische Schicht, insbesondere eine Kohlenstoff, ein Carbid, Nitrid oder Carbonitrid aufweisende Schicht, vorgesehen ist. Als solche ist eine Schicht aus Iridiumnitrid oder Iridiumoxid brauchbar, alternativ kann aber auch eine Unterlageschicht aus metallischem Iridium vorgesehen sein.

Die organische Schicht selbst kann eine in Kontakt mit dem Trägerkörper stehende und die Haftung an diesem vermittelnde erste Teilschicht aufweisen. Diese Teilschicht kann hinsichtlich der Reaktivität an den Trägerkörper angepaßt sein. Zur Erreichung einer spezifischen Funktionalisierung - insbesondere für zusätzliche Verwendungen der Elektrode - wird an die reaktiven Gruppen eine der gewünschten physiologischen Wirkung entsprechend gewählte Verbindung, insbesondere als Ligand, spezifisch gebunden. In speziellen Fällen sind beide Funktionen aber auch in einer homogenen Schicht realisierbar.

Von großer Bedeutung für den üblichen Einsatz einer Stimulationselektrode sind Ausbildungen, bei denen die äußere Oberfläche der organischen Schicht gegenüber der unspezifischen Adsorption von Bio-Makromolekülen passiviert ist - etwa indem sie Polyethylenglykol aufweist - oder bei denen sie Mittel zur Steuerung der Benetzbarkeit, insbesondere eine Moleküle mit einer OH-, COOH- oder NH2-Endgruppe aufweisende Verbindung zur Erhöhung der Benetzbarkeit oder eine eine CH3-Gruppe oder eine perfluorierte Alkylkette aufweisende Verbindung zur Verringerung der Benetzbarkeit, aufweist.

Vorteilhafte Weiterbildungen der Erfindung sind in den Unteransprüchen gekennzeichnet bzw. werden nachstehend zusammen mit der Beschreibung der bevorzugten Ausführung der Erfindung anhand der Figuren näher dargestellt. Es zeigen:
die Figuren 1a bis 1c eine Prinzipdarstellung des Aufbaus einer Polyelektrolyt-Multischicht,
die Figuren 2a bis 2c schematisch die chemische Struktur von einigen Verbindungen, die in Polyelektrolyt-Multischichten gemäß den Figuren 1a bis 1c eingesetzt werden,
die Figuren 3 und 3a eine schematische Querschnittsdarstellung des distalen Endes einer unipolaren Herzschrittmacher-Elektrodenleitung mit einem Elektrodenkopf gemäß einer Ausführungsform der Erfindung,
Figur 4 eine Prinzipdarstellung einer bipolaren Herzschrittmacher-Elektrodenanordnung, in der eine Ausführung der erfindungsgemäßen Stimulationselektrode eingesetzt ist,
Figur 5 eine Prinzipdarstellung zu einer Sensorelektrode nach einer weiteren Ausführungsform der Erfindung und
Figur 6 eine Prinzipdarstellung zu einer entzündungshemmenden Elektrode nach einer weiteren Ausführungsform der Erfindung.

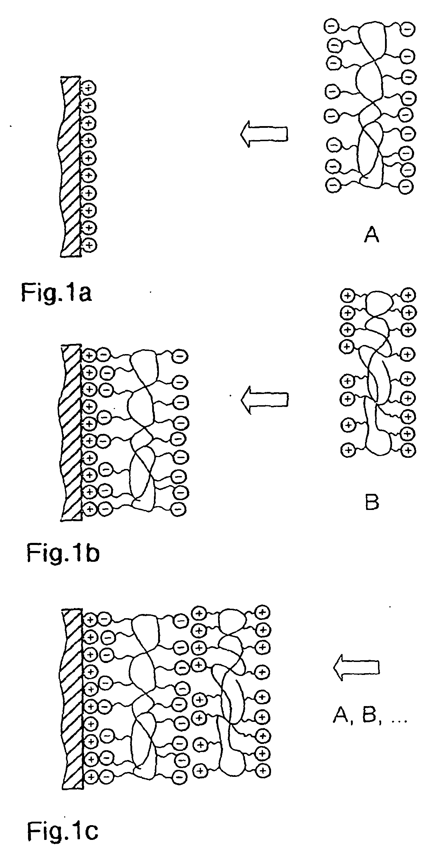

Die Figuren 1a bis 1c geben - in stark vereinfachter grafischer Darstellung der Verbindungen, die nicht deren tatsachliche räumliche Struktur in der Lösung oder nach der Adsorption wiedergeben soll - eine Prinzipdarstellung der ersten Schritte des Aufbaus einer Polyelektrolyt-Multischicht als organische Schicht.

An eine (etwa mittels Cysteamin) mit positiven Oberflächenladungen belegte metallische Oberfläche lagert sich, etwa beim Eintauchen in eine Lösung von Poly(styrensulfonat)-Natriumsalz (M = 100.000, Konzentration 1,01 x 10⁻² monomol/l, NaCl-Konzentration ca. 2,0 mol/l), zunächst -wie in Fig. 1a und 1b dargestellt - als anionischer Polyelektrolyt A Polystyrensulfonat (PSS) an, wobei die Schichtdicke nach ca. 20 min 45 A (4,5 nm) beträgt. Nach Abwaschen mit hochreinem Wasser wird das Substrat in eine zweite Lösung mit Poly(allylamin)-Hydrochlorid (M = 50.000 - 65.000, Konzentration 1,05 x 10⁻², NaCl-Konzentration ca. 2,0 mol/l) eingetaucht, die den kationischen Polyelektrolyten Poly(allylamin) (PAH) enthält, der sich - wie in Fig. 1b und 1c gezeigt - als Verbindung B anlagert. Hierbei beträgt nach ca. 20 min die Schichtdicke ca. 7 A (0,7 nm). Ein AB-Paar bzw. eine Wiederholeinheit der adsorbierten Struktur hat demnach eine "Gitterkonstante" von etwas über 50 A (5 nm), was durch Untersuchung der Kleinwinkel Röntgenstreuung bestätigt wurde. Wiederholungen der Eintauchvorgänge führen in analoger Weise zur Adsorption weiterer Schichten A und B bzw. weiterer Wiederholeinheiten. Bei den Untersuchungen der Erfinder erwiesen sich Schichtzahlen zwischen 30 und 40 als praktikabel. Die einzelnen Schichtdicken können über die Salz-Zugabe nach Bedarf gesteuert werden.

Als Verbindung A wurde auch erfolgreich Poly(vinylsulfat) (PVS) als Natriumsalz (M = 245.000) eingesetzt, wobei sich - unter spezieller Einstellung der Lösung mit PAH z.B. eine Paar-Schichtdicke von ca. 13,5 A (1,35 nm) ergab. Auch kombinierte Schichtaufbauten, beispielsweise aus mehreren PSS/PAH-Paaren und anschließend einigen PVS/PAH-Paaren sind in vorteilhafter Ausführung der Erfindung realisierbar.

Bei einer modifizierten Verfahrensführung wird - auf grundsätzlich dieselbe Weise wie oben beschrieben - als dritte Verbindung C noch Poly-(1-(4-(3-carboxy-4-hydroxyphenylazo)benzensulfonamid)-1,2-ethandiyl) (PAZO) als Natriumsalz (Konzentration 9,8 x 10⁻³ monomol/l, NaCl-Konzentration 0,1 mol/l) eingesetzt. Die Wiederholeinheit umfaßt hier folglich eine Abfolge ABCB.

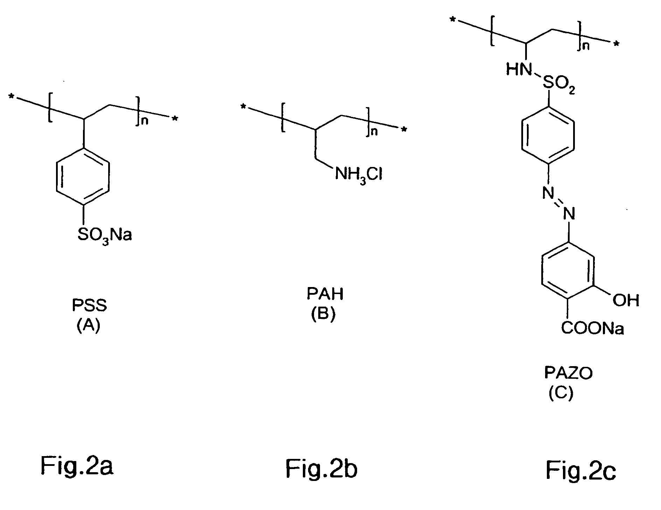

Die Figuren 2a bis 2c zeigen schematisch die chemische Struktur der oben genannten Verbindungen, die in Polyelektrolyt-Multischichten gemäß den Figuren 1a bis 1c eingesetzt werden,

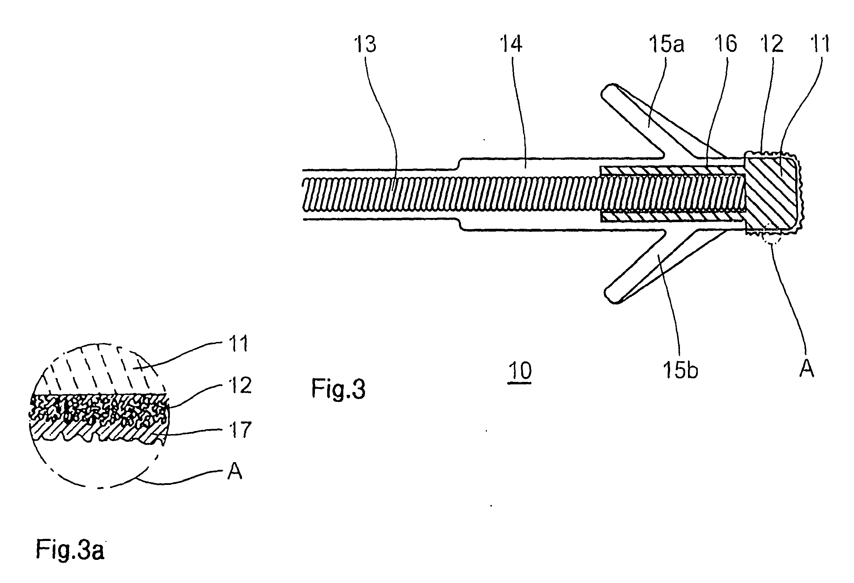

Die Figuren 3 und 3a zeigen in einer schematischen Querschnittsdarstellung das distale Ende einer unipolaren Herzschrittmacher-Elektrodenleitung 10. Es handelt es hier um eine unipolare Noppenelektrode mit einem einen zylinderförmigen Grundkörper 11 aus Titan aufweisenden Kopf. Der zylinderförmige Grundkörper 11 weist eine aus Iridiumnitrid (IrN) bestehende Oberflächenbeschichtung 12 auf, die mittels Kathodenzerstäubung (reaktives Sputtern) auf den Grundkörper aufgebracht ist. Die Elektrode weist eine gewendelte, elektrisch leitende Zuleitung 13 auf, die mit einer elektrisch isolierenden Ummantelung 5 aus Silikon versehen ist. An die Silikonummantelung angeformt sind nach rückwärts (proximal) gerichtete flexible Befestigungsele mente (Finnen) 15a und 15b, welche zur Fixierung der Elektrode im Herzen dienen, wobei die Oberfläche des Grundkörpers in Kontakt mit der Herzinnenwandung gehalten wird. Der Grundkörper 11 ist mittels eines hohlzylindrischen Ansatzes 16 über die Zuleitung 13 geschoben und dort befestigt.

In Figur 3a ist ein Ausschnitt der aktiven Oberfläche vergrößerten wiedergegeben. Wie aus der Darstellung ersichtlich ist, wird durch die (unmaßstäblich vergrößerte) fraktale räumliche Geometrie der im mikroskopischen Bereich stengel- bzw. blumenkohlartig gewachsenen Beschichtung 12 eine wesentliche Vergrößerung der aktiven Oberfläche erzielt. Die erzielte Oberflächenvergrößerung beträgt infolge geeigneter Wahl der Verfahrensparameter des Sputterverfahrens zwei bis über drei Größenordnungen, bezogen auf die Oberfläche eines glatten Zylinders mit den Abmessungen des Grundkörpers 11. An die IrN-Beschichtung 12 ist nach einem oben beschriebenen Verfahren eine organiche Schicht 17 mit wenigen Nanometern Schichtdicke adsorbiert, die die elektrischen Eigenschaften der Beschichtung 12 praktisch nicht beeinträchtigt, aber den Reizschwellenverlauf während des Einwachsens der Elektrode 10 an der Herzwand deutlich positiv beeinflußt.

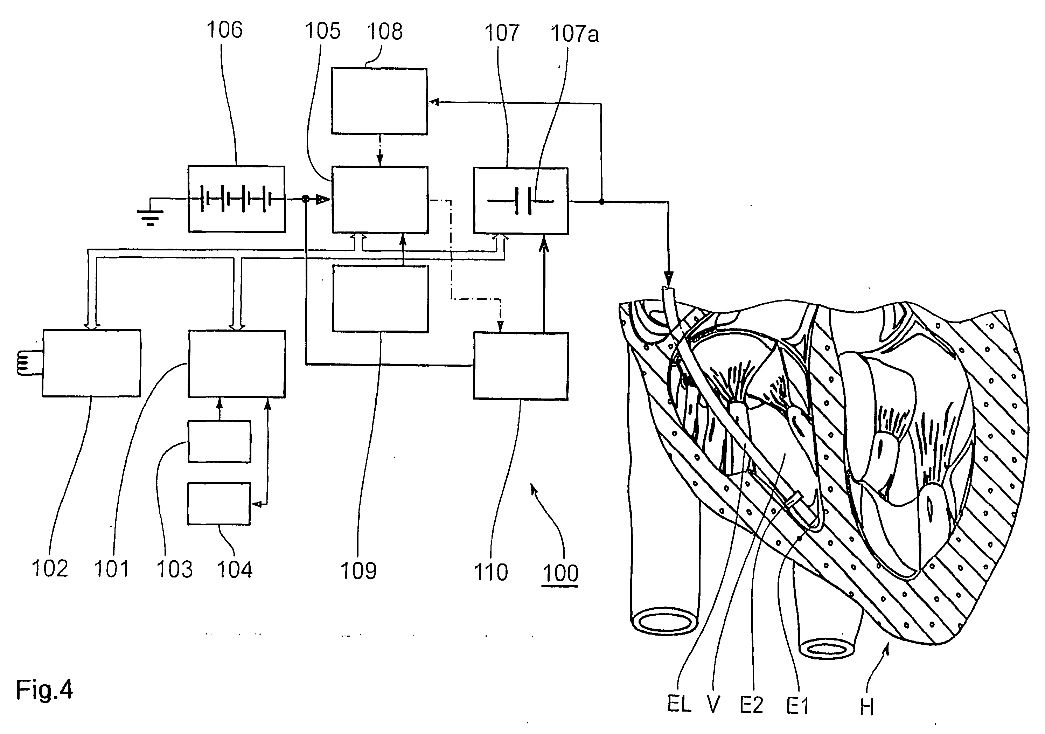

Figur 4 zeigt - in einer wiederum stark vereinfachten Darstellung - einen Einkammer-Bedarfsschrittmacher 100, der über eine bipolare Elektrodenleitung EL mit einer Spitzenelektrode E1 und einer Ringelektrode E2 einerseits zum Abfühlen von natürlichen Herzaktionen und zum anderen zur bedarfsweisen Abgabe von Stimulationsimpulsen mit der rechten Herzkammer V eines Herzens H verbunden ist.

Als wesentliche Steuerbaugruppen des Schrittmachers 100 sind ein Mikroprozessor 101, dem in üblicher Weise eine Telemetrieeinheit 102 sowie ein Programmspeicher 103 und ein Datenspeicher 104 zugeordnet sind, sowie eine Controller-/Zeitsteuereinheit 105 gezeigt. Eine Lithiumbatterie 106 dient zur Stromversorgung der Schrittmacherkomponenten und führt über eine Pumpsteuerschaltung 110 einer Ausgangsstufe 107 Stimulationsenergie zu. Die Ausgangsstufe umfaßt - wie durch den mit 107a bezeichneten Kondensator symbolisiert - eine Speicherkondensatoranordnung zur Speicherung elektrischer Energie zur Erreichung einer gegenüber der Batteriespannung erhöhten Stimulationsamplitude. Zwischen dem Mikroprozessor 101, der Telemetrieeinheit 102, der Controller-/Zeitsteuereinheit 105 und der Ausgangsstufe 107 ist ein Daten- und Steuersignalbus dargestellt. Mit den Elektroden E1, E2 ist in üblicher Weise eine Eingangsstufe 108 verbunden, die in Datensignalverbindung mit einem Steuereingang der Controller-/ Zeitsteuereinheit 105 steht. Dieser ist eingangsseitig ferner eine Oszillatorschaltung 109 zugeordnet. Ausgangsseitig ist die Controller/Zeitsteuerschaltung - neben den über die Busleitung realisierten Verbindungen - mit einem Steuereingang der Pumpsteuerschaltung 110 verbunden. Die Stimulationimpulserzeugung und -abgabe und die meisten weiteren Betriebsabläufe werden bei dieser Anordnung in an sich bekannter Weise gesteuert, so daß diesbezüglich keine spezielle Erläuterung erforderlich ist.

Die Ausführung der Elektroden E1, E2 mit einer organischen Schicht gemäß den weiter oben gegebenen Spezifikationen ermöglicht einen Betrieb des Schrittmachers mit im wesentlichen konstanter Abfühlempfindlichkeit und Stimulationsenergie auch während des Zeitraums des Einwachsens der Spitze der Elektrodenleitung EL in die Herzwandung. Die organische Beschichtung verhindert das Auftreten von Gewebsirritationen bzw. Entzündungen, die bei herkömmlichen Elektroden erhebliche temporäre Reizschwellenerhöhungen zur Folge haben können. Dadurch werden Neueinstellungen der Sensing-Verstärkung und/oder der Stimulationsamplitude verzichtbar und der Pumpstrom - und damit der Batterieverbrauch - in dieser Phase verringert, womit sich insgesamt ein vereinfachter Einsatz und höhere Lebensdauer des Schrittmachers ergeben.

Es besteht auch die Möglichkeit, durch Einbau geladener biologischer Moleküle in die organische Beschichtung - speziell als "Ersatz" für eine oder mehrere Schichten in einer Polyelektrolyt-Multischicht oder durch kovalente Ankopplung an reaktive Gruppen der Schicht über Seitengruppen der Moleküle - eine biologisch/physiologische Funktion zu realisieren und gleichzeitig die betreffenden Moleküle sicher zu immobilisieren.

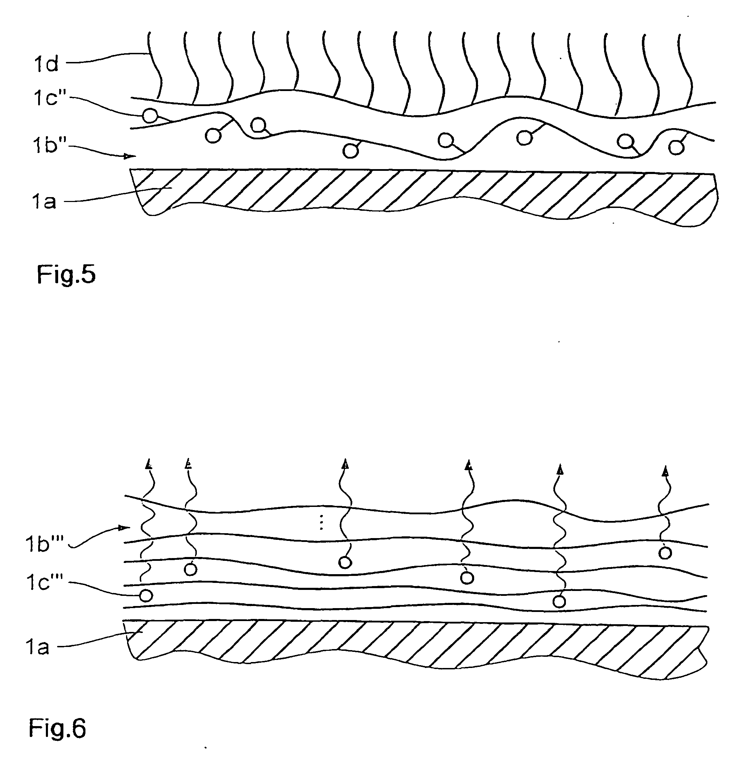

Dies ist beispielhaft in Fig. 5 skizziert, in der mit Ziffer 1a wiederum der Trägerkörper, mit 1b" eine Haftschicht, mit 1c" z.B. Enzym-Moleküle und mit 1d eine PEG-Terminierungsschicht bezeichnet sind. Ein Beispiel hierfür ist der Einbau von Glucoseoxidase zur Realisierung einer amperometrisch wirkenden Biosensorelektrode zur Glucosebestimmung.

Eine weitere Funktionalisierungsmöglichkeit stellt Fig. 6 dar, in der in Form einer Prinzipskizze die Einlagerung eines (als solchen bekannten) entzündungshemmenden Wirkstoffs 1c''' in eine relativ dicke Polyelektrolyt-Multischicht mit hoher Dielektrizitätskonstante 1b''' auf einer Elektrodenoberfläche 1a gezeigt ist. Der Austritt des Wirkstoffs aus der als Depot wirkenden organischen Schicht ist - wie mit den Pfeilen symbolisiert ist diffusionsgesteuert.

## Patentansprüche

1. Implantierbare Stimulationselektrode (1; 1'; 10; E1, E2) zur Verwendung mit einem implantierbaren Gewebestimulator (100), insbesondere Herzschrittmacher, Defibrillator, Knochen- oder Neurostimulator, mit einem metallischen Trägerkörper (1a, 1a'; 11) und einer auf diesen aufgebrachten Beschichtung (1b; 1b', 1c'; 1b", 1d; 1b"'; 12, 17) zur Verringerung der Elektrodenimpedanz und/oder zur Erhöhung der Gewebsverträglichkeit, wobei
eine im wesentlichen die gesamte äußere Oberfläche der Stimulationselektrode bildende, dünne, spezifisch funktionalisierte organische Beschichtung (1b; 1c'; 1b", 1d; 1b"'; 17) vorgesehen ist, die aufgrund irreversibler Physisorption oder kovalenter chemischer Bindung an der darunterliegenden Oberfläche fest haftet,
**dadurch gekennzeichnet daß** die organische Beschichtung einen durch sukzessive Adsorption eines anionischen und eines kationischen Polyelektrolyten (A, B) gebildeten mehrschichtigen Aufbau (1b"') aufweist.

2. Implantierbare Stimulationselektrode nach Anspruch 1, **dadurch gekennzeichnet daß** die organische Schicht (1b") Sondenmoleküle (1c"), insbesondere Enzymmoleküle, aufweist derart, daß die Stimulationselektrode als Biosensorelektrode wirken kann.

3. Implantierbare Stimulationselektrode nach Anspruch 1, **dadurch gekennzeichnet daß** die organische Schicht (1b"') einen medizinischen Wirkstoff (1c"'), insbesondere ein entzündungshemmendes Medikament, aufweist, der aus der organischen Schicht diffusions- oder lösungsgesteuert austragbar ist.

4. Implantierbare Stimulationselektrode nach Anspruch, **dadurch gekennzeichnet daß** der medizinische Wirkstoff (1c"') im wesentlichen zwischen Teilschichten des mehrschichtigen Aufbaus (1b"') eingebettet ist.

5. Implantierbare Stimulationselektrode nach Anspruch 1, **dadurch gekennzeichnet daß** die Schichtdicke der organischen Beschichtung im Bereich zwischen 1 und 100 nm liegt.

6. Implantierbare Stimulationselektrode nach Anspruch 1, **dadurch gekennzeichnet daß** die organische Beschichtung zwischen 10 und 50 Paare eines anionischen und eines kationischen Schichtbildners aufweist.

7. Implantierbare Stimulationselektrode nach Anspruch 1, **dadurch gekennzeichnet daß** die organische Beschichtung einander abwechselnde Schichten A aus Polystyrensulfonat bzw. Polystyrensulfonat-Natriumsalz oder Polyvinylsulfonat bzw. Polyvinylsulfonat-Kaliumsalz und B aus Poly-4-vinylbenzyl (N, N-diethyl-N-methyl)ammoniumjodid oder einem Polyallylamin bzw. Polyallylamin-Hydrochlorid aufweist.

8. Implantierbare Stimulationselektrode nach Anspruch 1, **dadurch gekennzeichnet daß** die organische Schicht (1b; 1b"') an der äußeren Oberfläche durch einen ungeladenen Polymeren (1c; 1d), insbesondere Polyethylenoxid oder Polyethylenglykol, terminiert ist.

9. Implantierbare Stimulationselektrode nach Anspruchs 2 und 8, **dadurch gekennzeichnet daß** die Sondenmoleküle (1c") im wesentlichen unterhalb des ungeladenen Polymeren (1d) in die organische Schicht eingebettet sind.

10. Implantierbare Stimulationselektrode nach Anspruch 1, **dadurch gekennzeichnet daß** die organische Beschichtung eine relative Dielektrizitätskonstante von größer als 100, insbesondere von größer als 300, aufweist.

11. Implantierbare Stimulationselektrode nach Anspruch 1, **dadurch gekennzeichnet daß** die darunterliegende Oberfläche eine derartige Oberflächenrauhigkeit bzw. -porosität aufweist, daß die effektive Physisorptions-Oberfläche um mindestens eine Größenordnung größer als die sich aus der geometrischen Grundform der Stimulationselektrode ergebende Oberfläche ist.

12. Implantierbare Stimulationselektrode nach Anspruch 11, **dadurch gekennzeichnet daß** die darunterliegende Oberfläche eine fraktale Oberflächengeometrie aufweist derart, daß die effektive Physisorptions-Oberfläche um zwei bis über drei Größenordnungen größer als die sich aus der geometrischen Grundform der Stimulationselektrode ergebende Oberfläche ist.

13. Implantierbare Stimulationselektrode nach Anspruch 1, **dadurch gekennzeichnet daß** zwischen dem metallischen Trägerkörper (11) und der organischen Beschichtung (17) eine nichtmetallische Schicht (12), insbesondere eine Kohlenstoff, ein Oxid, Carbid, Nitrid oder Carbonitrid aufweisende Schicht, vorgesehen ist.

14. Implantierbare Stimulationselektrode nach Anspruch 1, **dadurch gekennzeichnet daß** zwischen dem metallischen Trägerkörper (1a'; 11) und der organischen Schicht (1c'; 17) eine Schicht (1b'; 12) aus Iridium, Iridiumnitrid oder Iridiumoxid vorgesehen ist.

## Claims

1. Implantable stimulation electrode (1; 1'; 10; E1, E2) for use with an implantable tissue stimulator (1,00), in particular a pacemaker, defibrillator, bone or neurostimulator, comprising a metal substrate body (1a, 1a'; 11) and a coating (1b; 1b', 1c'; 1b'', 1d; 1b"'; 12, 17), applied to the substrate body, for reducing the electrode impedance and/or increasing the tissue compatibility, wherein a thin, specifically functionalised organic coating (1b; 1c'; 1b'', 1d; 1b"'; 17) substantially forming the entire outer surface of the stimulation electrode is provided, which adheres firmly to the underlying surface as a consequence of irreversible physisorption or covalent chemical bonding, **characterised in that** the organic coating has a multi-layer structure (1b"') formed by successive adsorption of one anionic and one cationic polyelectrolyte (A, B).

2. Implantable stimulation electrode according to claim 1, **characterised in that** the organic layer (1b'') has sensor molecules (1c"), in particular enzyme molecules, in such a way that the stimulation electrode can act as a biosensor electrode.

3. Implantable stimulation electrode according to claim 1, **characterised in that** the organic layer (1b"') has a medicinal active ingredient (1c"') in particular an antiinflammatory medication, which can be exported from the organic layer by diffusion control or solution control.

4. Implantable stimulation electrode according to claim 3, **characterised in that** the medicinal active ingredient (1c"') is substantially embedded between sub-layers of the multi-layer structure (1b''').

5. Implantable stimulation electrode according to claim 1, **characterised in that** the layer thickness of the organic coating is in the range between 1 and 100 nm.

6. Implantable stimulation electrode according to claim 1, **characterised in that** the organic coating has between 10 and 50 pairs of an anionic and a cationic layer former.

7. Implantable stimulation electrode according to claim 1, **characterised in that** the organic coating has alternating layers (A) made of polystyrene sulphonate or polystyrene sulphonate sodium salt or polyvinyl sulphonate or polyvinyl sulphonate potassium salt and B of poly-4-vinylbenzyl (N,N-diethyl-N-methyl) ammonium iodide or a polyallylamine or polyallylamine hydrochloride.

8. Implantable stimulation electrode according to claim 1, **characterised in that** the organic layer (1b; 1b''') is terminated on the outer surface by an uncharged polymer (1c; 1d), in particular polyethylene oxide or polyethylene glycol.

9. Implantable stimulation electrode according to claim 2 and 8, **characterised in that** the sensor molecules (1c") are embedded in the organic layer substantially underneath the uncharged polymer (1d).

10. Implantable stimulation electrode according to claim 1, **characterised in that** the organic coating has a relative dielectric constant of greater than 100, in particular greater than 300.

11. Implantable stimulation electrode according to claim 1, **characterised in that** the underlying surface has a surface roughness or surface porosity such that the effective physisorption surface is larger by at least one order of magnitude than the surface resulting from the geometric basic shape of the stimulation electrode.

12. Implantable stimulation electrode according to claim 11, **characterised in that** the underlying surface has a fractal surface geometry such that the effective physisorption surface is greater by two to more than three orders of magnitude than the surface resulting from the geometric basic shape of the stimulation electrode.

13. Implantable stimulation electrode according to claim 1, **characterised in that** a non-metallic layer (12), in particular a layer having carbon, an oxide, carbide, nitride or carbonitride, is provided between the metal substrate body (11) and the organic coating (17).

14. Implantable stimulation electrode according to claim 1, **characterised in that** a layer (1b'; 12) of iridium, iridium nitride or iridium oxide is provided between the metal substrate body (1a'; 11) and the organic layer (1c'; 17).

## Revendications

1. Électrode de stimulation implantable (1 ; 1' ; 10; E1, E2) en vue d'une utilisation avec un stimulateur de tissu implantable (100), en particulier un stimulateur cardiaque, un défibrillateur, un stimulateur osseux ou un neurostimulateur, muni d'un support métallique (1a, 1a' ; 11) et d'un revêtement appliqué sur celui-ci (1b ; 1b', 1c'; 1b", 1d ; 1b"' ; 12, 17) en vue de la réduction de l'impédance d'électrode et/ou de l'augmentation de la compatibilité du tissu, dans laquelle
est prévu un revêtement organique (1b ; 1c'; 1b", 1d ; 1b"' ; 17) spécifiquement fonctionnalisé mince formant sensiblement la totalité de la surface externe de l'électrode de stimulation, qui adhère à la surface sous-jacente en raison d'une physisorption irréversible ou d'une liaison chimique covalente,
**caractérisée en ce que** le revêtement organique présente une structure à couches multiples (1b"') formée par l'adsorption successive d'un polyélectrolyte anionique et d'un polyélectrolyte cationique (A, B).

2. Électrode de stimulation implantable selon la revendication 1, **caractérisée en ce que** la couche organique (1b"') présente des molécules de sonde (1c"), en particulier des molécules enzymatiques, de sorte que l'électrode de stimulation peut fonctionner comme électrode de biocapteur.

3. Électrode de stimulation implantable selon la revendication 1, **caractérisée en ce que** la couche organique (1b"') présente un principe actif médical (1c"'), en particulier un médicament anti-inflammatoire, qui peut être délivré depuis la couche organique par diffusion ou par solution.

4. Électrode de stimulation implantable selon la revendication 3, **caractérisée en ce que** le principe actif médical (1c" ') est inséré sensiblement entre les couches partielles de la structure à couches multiples (1b"').

5. Électrode de stimulation implantable selon la revendication 1, **caractérisée en ce que** l'épaisseur de couche du revêtement organique se trouve dans une plage comprise entre 1 et 100 nm.

6. Électrode de stimulation implantable selon la revendication 1, **caractérisée en ce que** le revêtement organique présente entre 10 et 50 paires d'une couche adsorbée anionique et d'une couche adsorbée cationique.

7. Électrode de stimulation implantable selon la revendication 1, **caractérisée en ce que** le revêtement organique présente des couches alternées A de polystyrène sulfonate ou de polystyrène sulfonate-sel de sodium ou de polyvinyl sulfonate ou de polyvinyl sulfonate-sel de potassium et B de poly-4-vinylbenzyl(N,N-diethyl-N-methyl)iodure d'ammonium ou d'un polyallylamine ou polyallylamine-hydrochlorure.

8. Électrode de stimulation implantable selon la revendication 1, **caractérisée en ce que** la couche organique (1b ; 1b" ') se termine à la surface externe par un polymère non chargé (1c ; 1d), en particulier de l'oxyde de polyéthylène ou du polyéthylène glycol.

9. Électrode de stimulation implantable selon les revendications 2 et 8, **caractérisée en ce que** les molécules de sonde (1c") sont noyées sensiblement sous le polymère non chargé (1d) dans la couche organique.

10. Électrode de stimulation implantable selon la revendication 1, **caractérisée en ce que** le revêtement organique présente une constante diélectrique relative supérieure à 100, en particulier supérieure à 300.

11. Électrode de stimulation implantable selon la revendication 1, **caractérisée en ce que** la surface sous-jacente présente une rugosité ou une porosité superficielle de sorte que la surface de physisorption effective est supérieure d'au moins un ordre de grandeur à la surface obtenue à partir de la forme fondamentale géométrique de l'électrode de stimulation.

12. Électrode de stimulation implantable selon la revendication 11, **caractérisée en ce que** la surface sous-jacente présente une géométrie superficielle fractale de sorte que la surface de physisorption effective est supérieure de deux jusqu'à plus de trois ordres de grandeur à la surface obtenue à partir de la forme fondamentale géométrique de l'électrode de stimulation.

13. Électrode de stimulation implantable selon la revendication 1, **caractérisée en ce qu'**une couche non métallique (12), en particulier une couche présentant du carbone, un oxyde, un carbure, un nitrure ou un carbonitrure, est prévue entre le support métallique (11) et le revêtement organique (17).

14. Électrode de stimulation implantable selon la revendication 1, **caractérisée en ce qu'**une couche (lb' ; 12) en iridium, nitrure d'iridium ou oxyde d'iridium, est prévue entre le support métallique (1a' ; 11) et la couche organique (1c' ; 17).
